# EUROPEAN PATENT APPLICATION

(11) **EP 3 594 173 A1**
(43) Date of publication of application: **15.01.2020**
(21) Application number: 18182943.3
(22) Date of filing: 11.07.2018
(51) Int. Cl.: B67D 1/00

(54) **FLOW TYPE CARBONISATION APPARATUS AND BEVERAGE DISPENSER MIT SUCH APPARATUS**

(71) Applicant: Riprup Company S.A., St. Peter Port GY1 1WQ (GG)
(72) Inventor: Bissen, Monique, 75175 Pforzheim (DE); Schucker, Josef, 6622 Ronco Sopra Ascona (CH); Braun, Benjamin, 75334 Straubenhardt (DE)
(74) Representative: Wittmann, Günther

(57) **Abstract**

Flow-type carbonator comprising:
- a liquid inlet 111 for feeding pressurized liquid;
- a liquid outlet 138 for discharging carbonated liquid;
- a gas inlet portion 110 located downstream of the liquid inlet 111; and
- a turbulence section 200 located downstream of the gas inlet portion 110 through which the pressurized liquid flows, when gas flows through the gas inlet portion 110;
- wherein the turbulence section 200 comprises at least one turbulence element 206a having an outer pipe portion 208a and an inner pipe portion 210a,
- wherein the outer pipe portion 208a-h is partially closed by a dividing wall 216a and the inner pipe portion 210a extends from the dividing wall 216a;
- wherein a recess 214a is formed between a portion of the inner pipe portion and outer pipe portion; and
- wherein the inner pipe portion 210a and the outer pipe portion 208a are in flow communication with the liquid inlet 111 and the liquid outlet 138.

## Description

The present invention discloses a flow-type carbonization apparatus and a beverage dispenser having such flow-type carbonization apparatus. A beverage dispenser outputs a beverage, such as water, into a glass or bottle of a user. Some user prefer carbonized beverage, such as carbonized water. Since water is supplied from a tap, a tank or a canister to the water dispenser in a non-carbonized way, the beverage dispenser must comprise a carbonization apparatus for delivering carbonized beverage.

### Prior art

A significant amount of beverage dispensers comprises a tank in which water is carbonized. The water has to stagnate in this tank for a significant amount of time. Stagnation is generally undesired, since germs may form during stagnation.

Flow-type carbonization based on Venturi nozzles is known, wherein a stream of carbon dioxide is introduced in a water stream.

WO 2012/123462 A1 discloses a flow type carbonization apparatus.

EP 0 322 925 A2 discloses a nozzle for injecting gas into a liquid.

The prior art flow type water carbonizer have a comparably low efficiency.

### Summary of the invention

It is an object of the present invention to provide a flow-type carbonization apparatus with an improved efficiency and a beverage dispenser having such carbonization apparatus.

The object of the present invention is achieved by a flow-type carbonization apparatus according to claim 1 and a beverage dispenser according to claim 14.

The invention discloses a flow-type carbonization apparatus comprising a liquid inlet for feeding pressurized liquid, a liquid outlet for discharging carbonized liquid, a gas inlet portion located downstream of the liquid inlet and a turbulence section located downstream of the gas inlet portion through which the pressurized liquid flows, when gas flows through the gas inlet portion. The turbulence section is in fluid communication with the liquid inlet and the liquid outlet. The turbulence section comprises at least one turbulence element having an outer pipe portion and an inner pipe portion. The outer pipe portion is partially closed by a dividing wall and an inner pipe portion extends from the partially open dividing wall. The inner pipe portion extends within the outer pipe portion. The inner pipe portion and the outer pipe portion are connected by the dividing wall. A recess is formed between a portion of the inner pipe portion and the outer pipe portion. The inner pipe portion and the outer pipe portion are in flow communication with the liquid inlet and the liquid outlet.

The inner pipe portion may extend upstream from the dividing wall into the recess formed by the outer pipe portion. Thereby, water flowing from a chamber formed by the outer pipe portion is formed into the inner pipe portion having a smaller diameter than the outer pipe portion. The inventors of the present invention assume without wishing to be bound to a specific theory that the carbon dioxide bubbles are fragmented at the edge of the orifice of the inner pipe portion extending upstream into the outer pipe portion and solved by the liquid.

In use the beverage flows through the inner pipe portion into a chamber formed by the outer pipe portion. Thereby, a part of the liquid injected by the inner pipe portion is directed to the recess formed between the inner pipe portion, the outer pipe portion and the dividing wall. The inventors of the present invention assume without wishing to be bound to a specific theory that at the edge of the orifice of the inner pipe portion protruding into the chamber formed by the outer pipe portion the carbon dioxide bubbles are fragmented and solved in a more efficient way in the beverage.

The inner pipe portion may extend downstream into the dividing wall into the recess formed by the outer pipe portion. Further, the recess around the inner pipe portion causes a turbulent flow supporting solving of the carbon dioxide in the liquid.

The outer pipe portion extends further from the separating wall than the inner pipe portion, such that the outer pipe portion may form a chamber in which the beverage flows from the inner pipe portion and/or from which the beverage may flow into the inner pipe portion.

In one embodiment, the turbulence section comprises a plurality of turbulence elements in serial connection. The beverage flows from the liquid inlet through the plurality of turbulence elements in a serial flow connection to the liquid outlet.

In a portion of the turbulence section the dividing walls of two adjacent turbulence elements may be located adjacent to each other. In another portion of the turbulence section the end portion of the outer pipe portions of two adjacent turbulence elements may be located adjacent to each other, wherein the end portions of the inner pipe portions face each other. The two outer pipe portions form a chamber into which the two inner pipe portions extend at opposite sides of the chamber from the respective dividing wall.

The outer pipe portion and the dividing wall of the turbulence element form a cylinder wherein the inner pipe portion forms an opening in the dividing wall.

The turbulence section may comprise a plurality of chambers that are in serial flow communication with an inlet of the turbulence section and an outlet of the turbulence section. The chambers are formed by the outer pipe portions. The chambers are separated by the dividing walls. Each inner pipe portion extends through a dividing wall into the adjacent chambers. Recesses are formed around an inner pipe portion extending into the outer pipe portion. Since a plurality of chambers and inner pipe portions are arranged in serial fluid communication, the efficiency of the flow-type carbonization apparatus is increased significantly. In one embodiment three to four chambers are preferred. Generally, a fifth chamber does not increase the achieved carbon dioxide concentration in the water significantly.

The distance between two orifices of opposing inner pipe portions facing each other may correspond to approximately 50% to approximately 150%, preferably to approximately 70% to approximately 125%, more preferred to approximately 100% to approximately 120% of the inner diameter of the outer pipe portion. The distance between two orifices of opposing inner pipe portions facing each other may correspond to approximately 50% to approximately 150%, preferably to approximately 75% to approximately 125%, more preferred to approximately 85% to approximately 115% of the length of a flow channel formed by the inner pipe portion extending in a first chamber and a second inner pipe portion extending in a second chamber adjacent to the first chamber. The diameter of the inner pipe portion may correspond to approximately 5% to approximately 30%, preferably to approximately 10% to approximately 25%, more preferred to approximately 15% to approximately 20% of the diameter of the outer pipe portion. The thickness of the wall of the inner pipe portion may correspond to approximately 50% to approximately 100%, preferably to approximately 65% to approximately 85%, more preferred to approximately 70% to approximately 75% of the diameter of the inner pipe portion. The inner pipe portion may extend from the dividing wall approximately 50% to approximately 400%, preferably approximately 100% to approximately 300%, more preferred approximately 150% to approximately 250% of the diameter of the inner pipe into the chamber.

The inner pipe portion has to be sharp edged at the orifice. Preferably, the orifice of the inner pipe portion is manufactured by drilling.

The distance between the orifices of opposing inner pipe portions facing each other ranges approximately from 3.5 mm to approximately 12 mm, preferably from approximately 4.5 mm to approximately 10 mm, more preferred from approximately 6 mm to approximately 8 mm. The length of a flow channel formed by a first inner pipe portion extending in a first chamber and a second inner pipe portion extending in a second chamber adjacent to the first chamber ranges from approximately 3.5 mm to approximately 12 mm, preferably from approximately 4.5 mm to approximately 10 mm, more preferred from approximately 6 mm to approximately 8 mm. The diameter of the inner pipe portion may range from approximately 0.5 mm to approximately 3 mm, preferably from approximately 0.7 mm to approximately 2 mm, more preferred from approximately 1 mm to approximately 1.5 mm. The thickness of the wall of the inner pipe portion ranges from approximately 0.3 mm to approximately 1.5 mm, preferably from approximately 0.5 mm to approximately 1 mm, more preferred from approximately 0.7 mm to approximately 0.8 mm. The inner pipe portion may extend from the dividing wall approximately 1 mm to approximately 3 mm, preferably approximately 1.5 mm to 2.5 mm, more preferred approximately 1.7 mm to approximately 2.2 mm into the chamber. The inner diameter of the outer pipe portion ranges between approximately 4 mm to approximately 10 mm, preferably between approximately 4 mm to approximately 8 mm, most preferred between approximately 5 mm to approximately 7 mm.

In one embodiment the amount of gas injected may be time modulated by activating a gas injector in the gas inlet portion over a time period varying depending on the set concentration of carbon dioxide in the water.

In one embodiment the gas inlet portion may comprise at least a first gas injector and a second gas injector for injecting gas into the gas inlet section, wherein the first gas injector causes a first gas output flow and the second gas injector causes a second gas output flow, wherein the second gas output flow is at least 50% larger, preferably 70% larger, more preferred between 80% and 120%, most preferred at least 80 % larger than the first gas flow. Thereby, the amount of gas injected into the liquid can be controlled over a wider range without requiring additional process time for carbonization. The flow-type carbonization apparatus may further comprise a carbonization controller adapted to control the first gas injector and the second gas injector, wherein if a low quantity of gas shall be fed into the liquid, only the first gas injector is activated, if a medium quantity of gas shall be fed into the liquid, only the second gas injector is activated and if a high quantity of gas shall be fed into the liquid the first gas injector and the second gas injector are activated. It is to be understood that the medium quantity of gas is larger than the low quantity of gas and the high quantity of gas is higher than the medium quantity of gas. The amount of carbon dioxide injected into the liquid may also be controlled by the time of activation of the first and/or second gas injector.

The invention also discloses a beverage dispenser comprising the flow-type carbonization apparatus disclosed above. The beverage dispenser comprises a liquid valve and/or a liquid pump adapted to control the flow of liquid through the flow-type carbonization apparatus. The beverage dispenser may further comprise a gas valve and/or a gas pump adapted to control the flow of gas into the gas inlet portion. The controller may be adapted to control the liquid valve and/or liquid pump and the gas valve and/or the gas pump. The controller may control the liquid valve and/or liquid pump and the gas valve and/or gas pump such that gas is fed into the gas inlet opening during flow of the liquid through the flow-type carbonization apparatus.

Preferably the flow of water is less than 1 I per minute, preferably between 0.5 I per minute to 1 I per minute. If the water to carbonize has a temperature of 2°C the carbon dioxide concentration of approximately 5 g/l can be achieved with the present carbonization apparatus. If the water has a temperature of 8°C a carbon dioxide concentration of approximately 4 g/l can be achieved with the inventive flow-type carbonization apparatus. This corresponds to an efficiency of approximately 60%. The water fed through the carbonization apparatus may have a pressure from approximately 3 bar to approximately 4 bar. Between the carbon dioxide tank and the first gas injector and/or the second gas injector a pressure reducing valve, particularly a pressure regulating valve can be located to control the pressure of the carbon dioxide in a controlled range. A preferred carbon dioxide pressure at the inlet of the first and/or second gas injector is approximately 5 bar to approximately 6 bar.

The controller of the beverage dispenser may also implement the carbonization controller.

The beverage dispenser may further comprise a tempering device arranged downstream of the gas injection portion and upstream of the turbulence section. Preferably, the tempering device is a flow-type tempering device. The liquid flow in the tempering device is not laminar but rather meander shaped which supports reducing the size of the carbon dioxide bubbles and thus solving the carbon dioxide in the liquid, such as water.

### Short description of the drawings

The invention is now described in further detail with reference to the accompanying drawings showing a non-limiting embodiment of the present invention, wherein:
Figure 1 is a schematic view of components of a beverage dispenser; and
Figure 2 is a schematic sectional view of a turbulence section according to the present invention.

### Detailed description of the invention

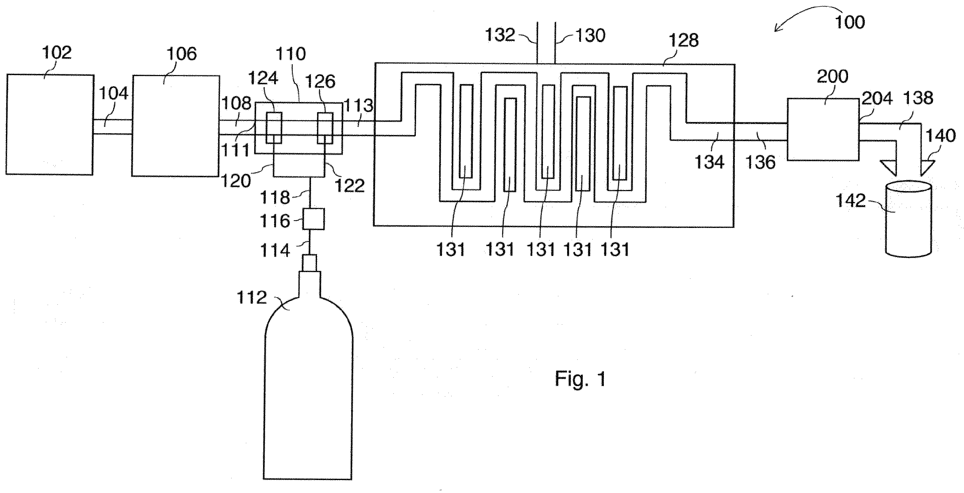

Reference is made to figure 1, showing a schematic view of a beverage dispenser 100 employing the present invention. The invention is described with reference to a water dispenser 100, but it is to be understood that the invention can be applied to any type of beverage dispenser. Reference numeral 102 indicates a water source. The water source may be a tap, a tank, a canister or the like. The water source 102 is connected by a pipe 104 to a pump 106. The pump 106 supplies water with a pressure of approximately 3 bar to approximately 4 bar into a pipe 108 connected to a gas inlet portion 110. The gas inlet portion 110 comprises a liquid inlet 111 for receiving pressurized water. The gas inlet portion 110 comprises a first gas injector 124 and a second gas injector 126. The second gas injector 126 can supply approximately twice as much carbon dioxide to the water flowing through the gas inlet portion as compared to the first gas injector 124.

The opening of the second gas injector may have a larger area as the opening of the first gas injector. The area of opening of the second gas injector may be two times larger as the area of the opening of the first gas injector. The area of the opening of the second gas injector may be at least 50 % larger, preferably 70 % larger, more preferred between 80 % and 120 % larger, most preferred at least 80 % larger than the area of the opening of the first gas injector.

The water dispenser comprises a carbon dioxide bottle 112 connected by a pipe 114 to a pressure reducing valve or pressure regulating valve 116. The pressure reducing valve 116 supplies carbon dioxide with a pressure of approximately 5 bar to approximately 6 bar to a pipe 118. The pipe 118 branches into a first injector supply pipe 120 and a second injector supply pipe 122. The first injector supply pipe 120 is connected to the first gas injector 124 and the second injector supply pipe 122 is connected to the second gas injector 126.

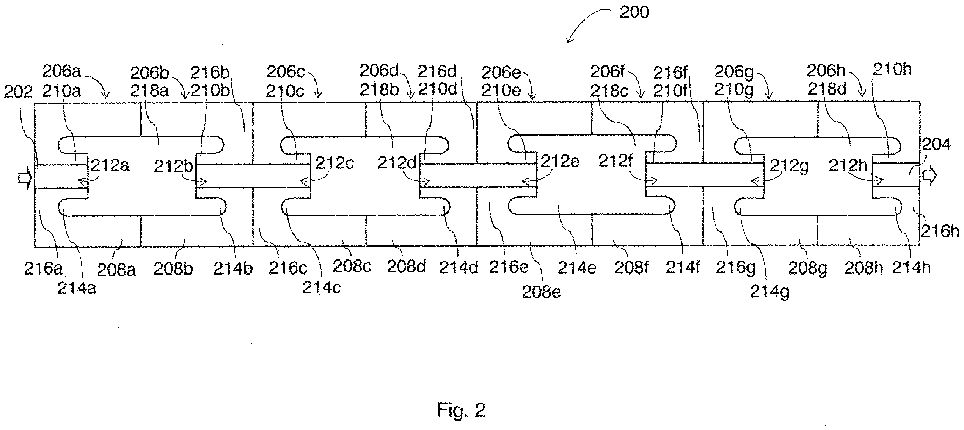

The gas inlet portion 110 is connected by an optional pipe 113 to a tempering device 128, i.e. a cooler. The water flows in the cooler through a meander-shaped pipe 134 which passes adjacent to cooling element 131. The cooling element 131 may comprise a Peltier element connected to a power supply 130, 132. The cooling element 131 may also be a heat exchanger through which a cooling media passes which is supplied by pipe 130 and discharged by pipe 132. The tempered water exits through an optional pipe 136 into a turbulence section 200 described in further detail with reference to figure 2.

The turbulence section 200 comprises an outlet 204 for outputting a carbonized water to a pipe 138 to which a nozzle 140 is connected dispensing the carbonized water into a vessel 142 of a user.

Reference is made to figure 2 showing a schematic cut away view of a turbulence section 200 according to the present invention. The turbulence section 200 comprises essentially four chambers 218a, 218b, 218c, 216d formed by outer pipe portions 208a-208h of a plurality of turbulence elements 206a-200h.

Into the first chamber 218a an inner pipe portion 210a of a first turbulence element 206a extends. Between the outer pipe portion 208a and the inner pipe portion 210a a recess 214a is formed. Between the outer pipe portion 208a and the inner pipe portion 210a a dividing wall 216a is arranged. The outer pipe portion 208a and the dividing wall 216a may form a cylinder, wherein the inner pipe portion 210a extends through the dividing wall 216a. The inner pipe portion 210a forms a fluid passage, wherein the fluid enters through the orifice 212a of the inner pipe portion 210a into the first chamber 216a. The outer pipe section 208a of the first turbulence element 206a extends further in the downstream direction as the inner pipe portion 210a of the first turbulence element 206a. The flow direction is indicated in Fig. 2 by arrows.

Adjacent to the first turbulence element 206a a second turbulence element 206b is located. The second turbulence element 206b is shaped essentially the same way as the first turbulence element 206a. Thus, for the sake of brevity, the second turbulence element is not described detail. The second turbulence element also comprises an outer pipe portion 208b connected by a dividing wall 216b with an inner pipe portion 210b. The second turbulence element 206b is arranged such in the turbulence section 200 that an orifice 212b of the inner pipe portion 216b of the second turbulence element 206b faces the orifice 212a of the inner pipe portion 210a of the first turbulence element 206a. The inner pipe portion 210b of the second turbulence element 206b extends upstream into the first chamber 218a.

The fluid enters through an orifice 212b in the inner pipe portion 210b of the second turbulence element 206b. The outer pipe portion 208b extends further from the divisional wall 216b in the upstream direction as the inner pipe portion 210b. Between the outer pipe portion 208b of the second turbulence element 206b and the inner pipe portion 210b a recess 214b is formed.

The combination of first turbulence element 206a and second turbulence element 206b can form in one embodiment a turbulence section having a single chamber 218a.

For increasing the efficiency of a plurality of a chambers 218a-218d and a plurality of turbulence elements 206a-206h can be arranged in serial flow communication.

In the embodiment disclosed in figure 2, adjacent to the second turbulence element 206b a third turbulence element 206c is arranged. The third turbulence element 206c is shaped essentially the same way as the first turbulence element 206a. A divisional wall 216c of the third turbulence element 206c is arranged adjacent (face-to-face) to the divisional wall 216b of the second turbulence element 206b. Thus, the inner pipe portion 210c of the third turbulence element extends downstream into the chamber 218b formed by the outer pipe portion 208c of the third turbulence element 206c. The fluid flows through the passage formed by the inner pipe portion 212b of the second turbulence element and the inner pipe portion 212c of the third turbulence element 206c and enters through the orifice 212c of the inner pipe portion 210c of the third turbulence element 206c into the chamber 218b. Between the outer pipe portion 208c and the inner pipe portion 210c a recess 214c is formed.

Adjacent to the third turbulence element 206c a fourth turbulence element 206d is located. The fourth turbulence element 206d is shaped essentially the same way as the first turbulence element 206a. Thus, for the sake of brevity, the fourth turbulence element is not described detail. The fourth turbulence element also comprises an outer pipe portion 208d connected by a dividing wall 216d with an inner pipe portion 210d. The fourth turbulence element 206d is arranged such in the turbulence section 200 that an orifice 212d of the inner pipe portion 210d of the fourth turbulence element 206d faces the orifice 212c of the inner pipe portion 210c of the third turbulence element 206c. The inner pipe portion 210d of the forth turbulence element 206d extends upstream into the second chamber 218b.

The fluid enters through an orifice 212d from the chamber 218b in the of the inner pipe portion 210d of the second turbulence element 206d. The outer pipe portion 208d extends further from the divisional wall 216b in downstream direction as the inner pipe portion 210d. Between the outer pipe portion 208d of the fourth turbulence element 206d and the inner pipe portion 210d a recess 214d is formed.

Adjacent to the fourth turbulence element 206d a fifth turbulence element 206e is arranged. The fifth turbulence element 206e is shaped essentially the same way as the first turbulence element 206a. A divisional wall 216e of the fifth turbulence element 206d is arranged adjacent (face-to-face) to the divisional wall 216d of the fourth turbulence element. Thus, the inner pipe portion 210e of the fifth turbulence element extends downstream into a third chamber 218c formed by the outer pipe portion 208e of the fifth turbulence element 206e. The fluid flows through the passage formed by the inner pipe portion 212d of the fourth turbulence element and the inner pipe portion 210e of the fifth turbulence element 206e and enters through the orifice 212e of the inner pipe portion 210e of the fifth turbulence element 206e into the chamber 218c. Between the outer pipe portion 208e and the inner pipe portion 210e a recess 214e is formed.

Adjacent to the fifth turbulence element 206e a sixth turbulence element 206f is located. The sixth turbulence element 206f is shaped essentially the same way as the first turbulence element 206a. The sixth turbulence element also comprises an outer pipe portion 208f connected by a dividing wall 216f with an inner pipe portion 210f. The sixth turbulence element 206f is arranged such in the turbulence section 200 that an orifice 212f of the inner pipe portion 216f of the sixth turbulence element 206f faces the orifice 212e of the inner pipe portion 210e of the fifth turbulence element 206e. The inner pipe portion 210f of the sixth turbulence element 206f extends upstream into the third chamber 218c.

Adjacent to the sixth turbulence element 206f a seventh turbulence element 206g is arranged. The seventh turbulence element 206g is shaped essentially the same way as the first turbulence element 206a. A divisional wall 216g of the seventh turbulence element 206g is arranged adjacent (face-to-face) to the divisional wall 216f of the sixth turbulence element. Thus, the inner pipe portion 210g of the seventh turbulence element extends downstream into a fourth chamber 218d formed by the outer pipe portion 208g of the seventh turbulence element 206g. The fluid flows through the passage formed by the inner pipe portion 212f of the sixth turbulence element 206f and the inner pipe portion 210g of the seventh turbulence element 206g and enters through the orifice 212g of the inner pipe portion 210g of the seventh turbulence element 206g into the fourth chamber 218d. Between the outer pipe portion 208g and the inner pipe portion 210g a recess 214g is formed.

Adjacent to the seventh turbulence element 206g an eighth turbulence element 206h is located. The eighth turbulence element 206h is shaped essentially the same way as the first turbulence element 206a. The eighth turbulence element 206h also comprises an outer pipe portion 208h connected by a dividing wall 216h with an inner pipe portion 210h. The eighth turbulence element 206h is arranged such in the turbulence section 200 that an orifice 212h of the inner pipe portion 216h of the eighth turbulence element 206h faces the orifice 212g of the inner pipe portion 210g of the seventh turbulence element 206g. The inner pipe portion 210h of the eighth turbulence element 206h extends downstream into the fourth chamber 218d.

The distance between the orifices 212a-212h of opposing inner pipe portions 210a-210h facing each other ranges approximately from 3.5 mm to approximately 12 mm, preferably from approximately 4.5 mm to approximately 10 mm, more preferred from approximately 6 mm to approximately 8 mm. The length of a flow channel formed by a first inner pipe portion 210a-210h extending in an upstream chamber 218a-218c and a second inner pipe portion 210a-210h extending in a downstream chamber 218b-218d adjacent to the first chamber ranges from approximately 3.5 mm to approximately 12 mm, preferably from approximately 4.5 mm to approximately 10 mm, more preferred from approximately 6 mm to approximately 8 mm. The diameter of the inner pipe portion 210a-210h may range from approximately 0.5 mm to approximately 3 mm, preferably from approximately 0.7 mm to approximately 2 mm, more preferred from approximately 1 mm to approximately 1.5 mm. The thickness of the wall of the inner pipe portion 210a-210h ranges from approximately 0.3 mm to approximately 1.5 mm, preferably from approximately 0.5 mm to approximately 1 mm, more preferred from approximately 0.7 mm to approximately 0.8 mm. The inner pipe portion 210a-210h may extend from the dividing wall 216a-216h approximately 1 mm to approximately 3 mm, preferably approximately 1.5 mm to 2.5 mm, more preferred approximately 1.7 mm to approximately 2.2 mm into the chamber. The inner diameter of the outer pipe portion 208a-208h ranges between approximately 4 mm to approximately 10 mm, preferably between approximately 4 mm to approximately 8 mm, most preferred between approximately 5 mm to approximately 7 mm.

The operation of the turbulence section is described below in more detailed. A fluid, in this embodiment the fluid comprising water and carbon dioxide, enters through the orifices 212a, 212c, 212e, 212g of the first, third, fifth and seventh turbulence element 206a, 206c, 206e, 206g into the respective chamber 218a, 218b, 218c, 218d. The inventors assume without wishing to be bound to a specific theory that at the orifice 212a, 212c, 212e, 212g the carbon dioxide bubbles are split up and distributed in the water and dissolve in the water. Further, the recess 214a, 214c, 214e, 214g around the inner pipe portion 210a, 210c, 210e, 210g forms a turbulent flow of the fluid in which the water can dissolve carbon dioxide in a particular efficient way.

Further, the recess 214a, 214c, 214e, 214g cause a particular turbulence flow in the chambers 218a, 218b, 218c, 218d contributing to solving carbon dioxide in water.

The fluid exits the chamber 218a, 218a, 218c, 218d by the orifice 212b, 212d, 212f, 212h of the inner pipe portion 210b, 210d, 210f, 210h of the second, fourth, sixth and eight turbulence element 206b, 206d, 206f, 206h, respectively. The inventors assume that at the edge of the orifice 212b, 212d, 212f, 212h the bubbles of carbon dioxide are divided and split up and dissolved more efficiently in the water. Further, the recess 214b, 214d, 214f, 214h between the outer pipe portion 206b, 206d, 206f, 206h and the inner pipe portion 216b, 216d, 216f, 216h increase the turbulence of the flow in the chamber 218a, 218b, 218c, 218d adding to the efficiency of the carbonization.

Preferably the flow of water through the turbulence section 200 is less than 1 I per minute, preferably between 0.5 I per minute to 1 I per minute. If the water to carbonite has a temperature of 2°C a carbon dioxide concentration of 5 g/l can be achieved with the present carbonization apparatus. If the water has a temperature of 8°C a carbon dioxide concentration of 4 g/l may be achieved with the inventive flow-type carbonization apparatus. This corresponds to an efficiency of approximately 60%. The water fed through the gas inlet portion 110 and/or the turbulence section 200 may have a pressure from approximately 3 bar to approximately 4 bar.

## Claims

1. Flow-type carbonization apparatus, comprising:
- a liquid inlet for feeding pressurized liquid;
- a liquid outlet for discharging carbonized liquid;
- a gas inlet portion located downstream of the liquid inlet; and
- a turbulence section located downstream of the gas inlet potion though which the pressurized liquid flows, when gas flows through the gas inlet portion;
- wherein the turbulence section comprises at least one turbulence element having an outer pipe portion and an inner pipe portion,
- wherein the outer pipe portion is partially closed by a dividing wall and the inner pipe portion extends from the dividing wall;
- wherein a recess is formed between a portion of the inner pipe portion and outer pipe portion; and
- wherein the inner pipe portion and the outer pipe portion are in flow communication with the liquid inlet and the liquid outlet.

2. Flow-type carbonization apparatus according to claim 1, wherein the inner pipe portion extends upstream from the dividing wall into the recess formed by the outer pipe portion.

3. Flow-type carbonization apparatus according to claim 1 or 2, wherein the inner pipe portion extends downstream from the dividing wall into the recess formed by the outer pipe portion.

4. Flow-type carbonization apparatus according to any one of claims 1 to 3, wherein the outer pipe portion extends further from the separating wall than the inner pipe portion.

5. Flow-type carbonization apparatus according to any one of claims 1 to 4, wherein the turbulence section comprises a plurality of turbulence elements in serial connection.

6. Flow-type carbonization apparatus according to claim 5, wherein the dividing walls of two adjacent turbulence elements are located adjacent to each other.

7. Flow-type carbonization apparatus according to claim 5 or 6, wherein the end portion of outer pipe portions of two adjacent turbulence elements are located adjacent to each other, wherein the end portions of the inner pipe portions face each other.

8. Flow-type carbonization apparatus according to any one of claims 1 to 7, wherein the outer pipe portion and the dividing wall of the turbulence element form a cylinder, wherein the inner pipe portion forms an opening in the dividing wall.

9. Flow-type carbonization apparatus according to any one of claims 1 to 8, wherein the turbulence section comprises a plurality of chambers that are in serial flow communication with an inlet of the turbulence section and an outlet of the turbulence section,
- wherein the chambers are formed by the outer pipe portions;
- wherein the chambers are separated by the dividing walls;
- wherein each inner pipe portion extends through a dividing wall into the adjacent chambers; and
- wherein the recess is formed around the inner pipe portion extending into the outer pipe portion.

10. Flow-type carbonization apparatus according to claim 9, wherein the turbulence section is **characterized by** at least one of the following:
- the distance between the orifices of opposing inner pipe portions facing each other corresponds to approximately 50 % to approximately 150 %, preferably to approximately 75 % to approximately 125 %, more preferred to approximately 100 % to approximately 120 % of the inner diameter of the outer pipe portion;
- the distance between two orifices of opposing inner pipe portions facing each other corresponds to approximately 50 % to approximately 150 %, preferably to approximately 75 % to approximately 125 %, more preferred to approximately 85 % to approximately 115 % of the length of a flow channel formed by a first inner pipe portion extending in a first chamber and a second inner pipe portion extending in a second chamber adjacent to the first chamber;
- the diameter of the inner pipe portion corresponds to approximately 5 % to approximately 30 %, preferably to approximately 10 % to approximately 25 %, more preferred to approximately 15 % to approximately 20 % of the diameter of the outer pipe portion;
- the thickness of the wall of the inner pipe portion corresponds to approximately 50 % to approximately 100 %, preferably to approximately 65 % to approximately 85 %, more preferred to approximately 70 % to approximately 75 % of the diameter of the inner pipe portion;
- the inner pipe portion extends from the dividing wall approximately 50 % to approximately 400 %, preferably approximately 100 % to approximately 300 %, more preferred approximately 150% to approximately 250% of the diameter of the inner pipe into the chamber.

11. Flow-type carbonization apparatus according to claim 9 or 10, wherein the turbulence section is **characterized by** at least one of the following:
- the distance between the orifices of opposing inner pipe portions facing each other ranges from approximately 3.5 mm to approximately 12 mm, preferably from approximately 4.5 mm to approximately 10 mm, more preferred from approximately 6 mm to approximately 8 mm;
- the length of a flow channel formed by a first inner pipe portion extending in a first chamber and a second inner pipe portion extending in a second chamber adjacent to the first chamber ranges from approximately 3.5 mm to approximately 12 mm, preferably from approximately 4.5 mm to approximately 10 mm, more preferred from approximately 6 mm to approximately 8 mm;
- the diameter of the inner pipe portion ranges from approximately 0.5 mm to approximately 3 mm, preferably from approximately 0.7 mm to approximately 2 mm, more preferred from approximately 1 mm to approximately 1.5 mm;
- the thickness of the wall of the inner pipe portion ranges from approximately 0.3 mm to approximately 1.5 mm, preferably from approximately 0.5 mm to approximately 1 mm, more preferred from approximately 0.7 mm to approximately 0.8 mm;
- the inner pipe portion extends from the dividing wall approximately 1 mm to approximately 3 mm, preferably approximately 1.5 mm to 2.5 mm, more preferred approximately 1.7 mm to approximately 2.2 mm into the chamber;
- the inner diameter of the outer pipe portion ranges between approximately 4 mm to approximately 10 mm, preferably between approximately 4 mm to approximately 8 mm, most preferred between approximately 5 mm to approximately 7 mm.

12. Flow-type carbonization apparatus according to any one of claims 1 to 11, wherein the gas inlet portion comprises at least a first gas injector and a second gas injector for injecting gas into the gas inlet section, wherein the first gas injector causes a first gas output flow and the second gas injector causes a second gas output flow, wherein the second gas output flow is at least 50 % lager, preferably 75 % larger, more preferred between 80 % and 120 % larger, most preferred at least 80 % larger than the first gas flow.

13. Flow-type carbonization apparatus according to claim 12, further comprising a carbonization controller adapted to control the first gas injector and the second gas injector, wherein if a low quantity of gas shall be fed into the liquid, only the first gas injector is activated, if a medium quantity of gas shall be fed into the liquid, only the second gas injector is activated and if a high quantity of gas shall be fed into the liquid, the first gas injector and the second gas injector are activated.

14. Beverage dispenser comprising the flow-type carbonization apparatus according to any one of claims 1 to 13, further comprising
- at least one of a liquid valve and a liquid pump adapted to control the flow of liquid through the flow-type carbonization apparatus;
- at least one of a gas valve and a gas pump adapted to control the flow of gas into the gas inlet portion; and
- a controller adapted to control the at least one of the liquid valve and the liquid pump and the at least one of the gas valve and the gas pump, wherein the controller controls the at least one of the liquid valve and the liquid pump and the at least one of the gas valve or gas pump such that gas is fed into the gas inlet opening during flow of the liquid through the flow-type carbonizing apparatus.

15. Beverage dispenser according to claim 14, further comprising a tempering device arranged downstream of the gas injection portion and upstream of the turbulence section.
